# EUROPÄISCHE PATENTSCHRIFT

(11) **EP 1 294 938 B1**
(45) Veröffentlichungstag und Bekanntmachung des Hinweises auf die Patenterteilung: **26.12.2007**
(21) Anmeldenummer: 01929568.2
(22) Anmeldetag: 17.04.2001
(51) Int. Cl.: C12Q 1/68, C07H 21/04, C07H 21/00

(54) **ZELLBINDENDE NUKLEINSÄUREMOLEKÜLE (APTAMERE)**
CELL-BINDING NUCLEIC ACID MOLECULES (APTAMERS)
MOLECULES D'ACIDE NUCLEIQUE (APTAMERES) SE LIANT A DES CELLULES

(30) Priorität: 18.04.2000 DE 10019154
(43) Veröffentlichungstag der Anmeldung: 26.03.2003
(73) Patentinhaber: Eberhard-Karls-Universität Tübingen Universitätsklinikum, 72076 Tübingen (DE)
(72) Erfinder: SCHLÜSENER, Hermann, 72076 Tübingen (DE); BLANK, Michael, 70176 Stuttgart (DE)
(74) Vertreter: Otten, Hajo
(86) Internationale Anmeldenummer: PCT/EP2001/004340
(87) Internationale Veröffentlichungsnummer: WO 2001/079538

(56) Entgegenhaltungen:
- WO-A-96/34875
- WO-A-99/27138
- US-A- 6 013 443
- ALLIEGRO MARK C ET AL: "A nuclear protein regulated during the transition from active to quiescent phenotype in cultured endothelial cells." DEVELOPMENTAL BIOLOGY, Bd. 174, Nr. 2, 1996, Seiten 288-297, XP002202418 ISSN: 0012-1606
- NOBILE VALENTINA ET AL: "Inhibition of human angiogenin by DNA aptamers: Nuclear colocalization of an angiogenin-inhibitor complex." BIOCHEMISTRY, Bd. 37, Nr. 19, 12. Mai 1998 (1998-05-12), Seiten 6857-6863, XP002202419 ISSN: 0006-2960
- ALLIEGRO M C: "A C-terminal carbohydrate-binding domain in the endothelial cell regulatory protein, pigpen: new function for an EWS family member." EXPERIMENTAL CELL RESEARCH. UNITED STATES 15 MAR 2000, Bd. 255, Nr. 2, 15. März 2000 (2000-03-15), Seiten 270-277, XP002202420 ISSN: 0014-4827
- WILLIS M C ET AL: "LIPOSOME-ANCHORED VASCULAR ENDOTHELIAL GROWTH FACTOR APTAMERS" BIOCONJUGATE CHEMISTRY, AMERICAN CHEMICAL SOCIETY, WASHINGTON, US, Bd. 9, Nr. 5, 1. September 1998 (1998-09-01), Seiten 573-582, XP000778000 ISSN: 1043-1802
- PLATE KARL H: "Mechanisms of angiogenesis in the brain." JOURNAL OF NEUROPATHOLOGY & EXPERIMENTAL NEUROLOGY, Bd. 58, Nr. 4, April 1999 (1999-04), Seiten 313-320, XP002202421 ISSN: 0022-3069

## Beschreibung

Die vorliegende Erfindung betrifft Nukleinsäuremoleküle, die selektiv an Endothelzellen binden oder diese markieren, Verfahren zum Nachweisen einer Tumorerkrankung, die in den Verfahren verwendeten Nukleinsäuremoleküle, sowie die Verwendung der Nukleinsäuremoleküle.

Solche Verfahren, sowie die darin verwendeten Nukleinsäuremoleküle, die auch als Aptamere bezeichnet werden, sind bekannt. In Osborne et al., "Aptamers as therapeutic and diagnostic reagents: problems and prospects", Curr. Opin. Chem. Biol. 1997, 1: 5-9, befindet sich ein entsprechender Überblick.

Aptamere sind hochaffine RNA- und DNA-Oligo- bzw. Polynukleotide, die aufgrund ihrer spezifischen räumlichen Struktur eine hohe Affinität zu einem Zielmolekül besitzen. Um solche Aptamere zu finden, wird häufig ein kombinatorischer Ansatz genutzt, bei dem nach dem Zufallsprinzip eine große Zahl von Oligonukleotiden unterschiedlichster Sequenz und Sekundärstruktur enzymatisch erzeugt wird, aus denen Oligonukleotide mit hoher Affinität zu einem Zielmolekül herausgesucht und angereichert werden. Diese Technik wird auch SELEX ("systematic evolution of ligands by exponential enrichment") genannt.

Bei Kenntnis der Primärstruktur eines solchen Oligo-/Polynukleotids (Aptamers), kann dieses auch synthetisiert werden, z.B. durch Verwendung eines DNA-/RNA-Synthesizers.

Zielstrukturen für Aptamere können Rezeptoren und andere Proteine, Lipid- oder Kohlenhydratstrukturen sein. Es wurden auch schon hochaffine Aptamere gegen kleine Moleküle wie gegen Theophylin, siehe Jenison et al., "High-resolution molecular discrimination by RNA", Science 1994; 263 (5152): 1425-9, gegen ATP von Tang et al. "Rational design of allosteric ribozymes", Chem. Biol. 1997; 4 (6): 453-9, und gegen Arginin gefunden, siehe Geiger et al., "RNA aptamers that bind L-arginine with sub-micromolar dissociation constants and high enancio-selectivity" Nucl. Acids Res. 1996; 24 (6): 1029-36. Ebenso konnten proteinbindende Aptamere, die in ihrer natürlichen Umgebung normalerweise keine Nukleinsäuren binden, gefunden werden. Morris et al., "High affinity ligands from in vitro selection: Complex targets"; Proc. Natl. Acad. Sci. USA 1998; 95: 2902-2907, gelangen die Entwicklung von Aptameren, die gegen Zellmembranen gerichtet sind.

Nachteilig bei dem derzeitigen Stand der Technik ist, daß durch die Autoren der zitierten Publikationen keine vollständigen Sequenzen der Nukleinsäuremoleküle bereitgestellt werden, die an komplexes biologisches Material binden oder dieses markieren. Morris et al., die sich mit der Markierung von höhermolekularen Strukturen beschäftigen, geben lediglich Sequenzmotive an. Nukleinsäuremoleküle, die solche Sequenzmotive enthalten, binden gemäß dieser Veröffentlichung an künstlich erzeugte Vesikel aus Erythrozytenzellmembranen (Erythrozyten-Ghosts).

Aus der US 6,013,443 sind Nukleinsäuremoleküle bekannt, die an Transportermoleküle der Bluthirnschranke binden.

Aus der Publikation von Willis et al., "Liposome-Anchored Vascular Endothelial Growth Factor Aptamers", Bioconjugate Chem. 1998; 9: 573-82, ist ein Nukleinsäuremolekül bekannt, das an den vaskulären Endothelwachstumsfaktor (VEGF) binden kann.

Aus der WO 96/34875 A1 sind Nukleinsäuremoleküle bekannt, die an Erythrozyten-Ghosts sowie an Glioblastomzellen binden können.

Aus der WO 27138 A1 sind Nukleinsäuremoleküle bekannt, die an Blutgefäße binden können.

Vor diesem Hintergrund ist es Aufgabe der vorliegenden Erfindung, ein weiteres Nukleinsäuremolekül der eingangs genannten Art bereitzustellen, insbesondere ein solches, das in der Lage ist, selektiv an Endothelzellen zu binden oder diese zu markieren.

Erfindungsgemäß wird diese Aufgabe durch ein Nukleinsäuremolekül mit der Nukleotidsequenz SEQ ID Nr. 9 aus dem beiliegenden Sequenzprotokoll gelöst.

Die der Erfindung zugrunde liegende Aufgabe wird dadurch vollkommen gelöst. Die Erfinder haben nämlich erkannt, daß ein Nukleinsäuremolekül mit der vorstehenden Nukleotidsequenz natives biologisches Material, nicht lediglich durch technische Methoden erzeugte Teile eines solchen, und speziell Endothelzellen erkennt und bindet. Nukleinsäuremoleküle, die die aufgeführte Sequenz enthalten, zeichnen sich nach Erkenntnis der Erfinder durch hohe Spezifität für das verwendete biologische Material aus.

Das Nukleinsäuremolekül ist ein Nukleinsäuremolekül mit der Nukleotidsequenz SEQ ID Nr. 9 aus dem beiliegenden Sequenzprotokoll.

Aus den Daten der Erfinder hat sich ergeben, daß bei Verwendung eines Nukleinsäuremoleküls mit der beanspruchten Nukleotidsequenz in dem eingangs erwähnten Verfahren die zugrunde liegende Aufgabe der Erfindung vollständig gelöst wird. Überraschenderweise sind die durch die Nukleotidsequenz vorgegebenen Sekundärstrukturen der Nukleinsäuremoleküle ausreichend, um eine hochspezifische Bindung an das biologische Material zu vermitteln.

In dem Nukleinsäuremolekül kann in dessen nicht-funktionellen Abschnitten zumindest ein Nukleotid ersetzt sein oder fehlen.

Die Erfinder haben nämlich erkannt, daß für eine selektive Bindung an eine biologische Struktur bestimmte funktionelle Bereiche innerhalb einer Sequenz des spezifischen Nukleinsäuremoleküls verantwortlich sind. Diese Bereiche liegen bei den zu Sekundärstrukturen gefalteten Sequenzen in den sogenannten "hairpin loops" oder "bulgs". Bei Übereinstimmung der Basensequenzen innerhalb dieser funktionellen Bereiche können zwei Nukleinsäuremoleküle bzw. Aptamere, die sich in ihrer Sequenz in nicht funktionellen Abschnitten voneinander unterscheiden, an die gleiche Struktur binden. Solche Unterscheidungen umfassen sowohl Nukleotidaustausche als auch Deletionen einzelner oder mehrerer Nukleotide, sowie Strangverkürzungen oder terminale Veränderungen.

Gegenstand der vorliegenden Erfindung ist auch ein Verfahren, in dem selektiv Endothelzellen gebunden oder markiert werden, worin hierfür ein Nukleinsäuremolekül verwendet wird, das die Nukleotidsequenz SEQ ID Nr. 9 enthält, wobei auch hier hinsichtlich dieser Sequenz Sequenzvariationen möglich sind, bei denen in den nicht-funktionellen Abschnitten des Nukleinsäuremoleküls zumindest ein Nukleotid ersetzt ist oder fehlt.

Die Sequenzen SEQ ID Nr. 1 bis SEQ ID Nr. 21 binden spezifisch an Endothelzellen. Diese, die luminale Seite von Blutgefäßen auskleidenden Zellen spielen unter anderem eine wichtige Rolle in der Vaskulogenese, Angiogenese und der Transcytose von Stoffen über Blut-Gewebebarrieren. In diesem Zusammenhang ist das Verfahren besonders zum Studium der Differenzierung von Endothelzellen sowie zum Markieren von tumorversorgenden Blutgefäßen oder entzündlichen endothelzellspezifischen Läsionen geeignet.

Bevorzugt ist es auch, wenn das Nukleinsäuremolekül an ein Therapeutikum gekoppelt ist.

Die beanspruchten Nukleinsäuremoleküle haben das Potential, als zielgerichteter Carrier für den Transport von Therapeutika an ihren Zielort zu fungieren. Durch solch einen zielgerichteten Einsatz von Therapeutika erhoffen sich die Erfinder vor allem eine Reduzierung der Dosierung bzw. eine drastische Verringerung unerwünschter Nebenwirkungen, da durch solch ein "Drug-Targeting" die ungewollte Wechselwirkung mit nicht-pathogenen Strukturen weitgehend vermieden wird.

Als Zielstrukturen kommen besonders tumoröse Gewebe in Betracht. Durch neuere Studien aus der Krebsforschung hat sich nämlich ergeben, daß die Unterbrechung der Blutversorgung eines Tumors ein vielversprechender Ansatz für eine Therapie darstellt (Übersichtsartikel in Cheresh, D. A., "Death to a blood vessel, death to a tumor", Nat. Med. 1998, 4 (4):395-6; Molema et al., "Rocking the foundations of solid tumor growth by attacking the tumor's blood supply", Immunol. Today 1998, 19 (9):392-4). Auch ein solches Verfahren läßt sich mit den neuen Nukleinsäuresequenzen vorteilhaft realisieren.

Während der Studien, die zu der vorliegenden Erfindung führten, haben die Erfinder herausgefunden, daß mittels eines Nukleinsäuremoleküls, das die Nukleotidsequenz SEQ ID Nr. 9 aus dem beiliegenden Sequenzprotokoll enthält, selektiv tumorspezifische Strukturen erkannt werden. Dadurch wird mittels dieses Nukleinsäuremoleküls eine hochspezifische Diskriminierung entarteten Tumorgewebes von gesundem Gewebe möglich. Die Erfinder stellen somit ein Werkzeug bereit, durch dessen Verwendung eine zuverlässige Basis für eine entsprechende zielgerichtete Therapie geschaffen wir.

In diesem Zusammenhang ist Gegenstand der vorliegenden Erfindung auch die Verwendung eines solchen Nukleinsäuremoleküls, das die Nukleotidsequenz SEQ ID Nr. 9 aus dem beiliegenden Sequenzprotokoll enthält oder daraus besteht, zum Nachweis einer Tumorerkrankung, sowie ein entsprechendes Verfahren zum Nachweisen einer Tumorerkrankung endothelialen Ursprungs in der Ratte, mit den Schritten:
a) Bereitstellen von biologischem Material,
b) Nachweis eines Tumormarkers, wobei der Nachweis mit einem Nukleinsäuremolekül erfolgt, das die Nukleotidsequenz SEQ ID Nr. 9 aus dem beiliegenden Sequenzprotokoll enthält oder daraus besteht.

Weitergehende Studien der Erfinder haben ergeben, daß durch das Nukleinsäuremolekül mit der Nukleotidsequenz SEQ ID Nr. 9 aus dem beiliegenden Sequenzprotokoll die Diskriminierung von Tumorgewebe gegenüber gesundem Gewebe über das Homolog zu dem Maus-/Rinder-Protein 'pigpen' erfolgt. Bei diesem Protein handelt es sich um ein bisher relativ unbekanntes endotheliales Protein mit einem Molekulargewicht von 67 kDa. Auch dessen Assoziation mit Tumorgewebe war bis dato unbekannt. So wurde dieses Protein bisher als ein kohlenhydratbindendes Protein mit RNA-Bindungs- und Transaktivierungsdomänen beschrieben, das möglicherweise mit der Kernmembran assoziiert ist; siehe z.B. Alliegro et al., "Protein Heterogeneity in the Coiled Body Compartment", Experimental Cell Research 239, 60-68 (1998).

Vor diesem Hintergrund ist Gegenstand der vorliegenden Erfindung sowohl die Verwendung eines Nukleinsäuremoleküls, das die Nukleotidsequenz SEQ ID Nr. 9 aus dem beiliegenden Sequenzprotokoll enthält, zum Nachweisen einer Tumorerkrankung endothelialen Ursprungs in der Ratte, wobei der Nachweis über das Protein 'pigpen' erfolgt, als auch allgemein ein Verfahren zum Nachweisen einer Tumorerkrankung, wobei der Nachweis über das menschliche Homolog zu dem Maus-/Rinder-Protein 'pigpen' erfolgt.

Des weiteren besteht eine Anwendung der beanspruchten Nukleinsäuremoleküle in dem Transport von Therapeutika an entzündliche endotheliale Läsionen. Mögliche Therapeutika sind deshalb z.B. antiangiogenetische oder antiinflamatorische Substanzen.

Durch Verlängerung der beanspruchten spezifischen Nukleinsäuremoleküle mit funktionstragenden DNA-Segmenten können lokal wirksame Immuntherapeutika hergestellt werden. Hier ist besonders an die Verwendung sogenannter CpG-Oligos gedacht, die profunde immunstimulierende Aktivität haben.

Die Erfinder der vorliegenden Anmeldung haben erstmals erkannt, daß die beanspruchten Nukleinsäuremoleküle zur Therapie der erwähnten Krankheitsbilder verwendet werden können.

Besonders bevorzugt ist es auch, wenn das Nukleinsäuremolekül an ein Diagnostikum gekoppelt ist.

Solch modifizierte Nukleinsäuremoleküle bzw. Aptamere haben gegenüber den in der Diagnostik und auch in dem oben erwähnten "Drug-Targeting" üblicherweise verwendeten monoklonalen Antikörpern eine Vielzahl von Vorteilen. Aufgrund der sequenzbedingten Ausbildung von Sekundärstrukturen ist das Repertoire an potentiell bindenden Liganden wesentlich größer als das verfügbare Immunrepertoire zur Herstellung von monoklonalen Antikörpern.

Des weiteren sind Aptamere wesentlich schneller und kostengünstiger zugänglich. Innerhalb von drei bis vier Wochen lassen sich Millionen potentieller Liganden untersuchen. Im Gegensatz hierzu muß die Herstellung von antikörperproduzierenden Hybridomzellen über Impfungen von Versuchstieren verlaufen. Diese Impfungen dauern mehrere Wochen. Zusätzlich sind Monate erforderlich, um einige hundert Hydridomzellinien zu untersuchen.

Da im Organismus keine extrazellulären Oligo-/Polynukleotide bekannt sind, die aufgrund ihrer Struktur Funktionen ausüben, haben solche Nukleinsäuremoleküle bzw. Aptamere außerdem das Potential "ungewöhnliche" Epitope zu erkennen, die sich der Erkennung durch Antikörper und somit dem "üblichen" Immunsystem entziehen.

Ein besonderer Vorteil der beanspruchten Nukleinsäuremoleküle besteht darin, daß diese die Bindungsspezifität und Affinität von guten monoklonalen Antikörpern erreichen. Sie sind somit besonders geeignet, in einem Diagnoseverfahren oder einem Verfahren zum "Drug-Targeting" eingesetzt zu werden. Hinsichtlich eines Diagnoseverfahrens wird vor allem an ein Verfahren gedacht, bei dem das Diagnostikum außerhalb des menschlichen Körpers verwendet wird.

Im Rahmen eines diagnostischen Verfahrens eignen sich als Diagnostikum besonders fluoreszierende Verbindungen, z.B. Fluorescinisothiocyanat (FITC), Biotin, Digoxigenin und deren Abkömmlinge, enzymatische Marker, Infrarot-Marker und Chelatbildner. Deshalb ist besonders ein Verfahren bevorzugt, bei dem das an das Nukleinsäuremolekül gekoppelte Diagnostikum aus der Gruppe ausgewählt ist, die die vorstehenden Substanzen enthält.

Bestandteil der Erfindung ist des weiteren die Verwendung der vorstehenden Nukleinsäuremoleküle für histologische Untersuchungen von Gewebeschnitten.

Die Erfinder haben erkannt, daß der direkte Einsatz der beanspruchten Nukleinsäuremoleküle als histologisches Agens möglich ist, was eine *in situ*-Lokalisation der biologischen Zielstruktur innerhalb des Gewebes erlaubt. Der besondere Vorteil solch einer Verwendung liegt in der eingangs erwähnten Spezifität der beanspruchten Nukleinsäuremoleküle für komplexes biologisches Material, vorzugsweise Endothelzellen. Dies ermöglicht eine gezielte Anfärbung dieser Strukturen ohne störende Hintergrundsignale oder unspezifische Kreuzreaktionen.

Besonders bevorzugt ist die Verwendung der vorstehenden Nukleinsäuremoleküle in einem der beschriebenen Verfahren, wenn die Nukleinsäuremoleküle selektiv an natives komplexes biologisches Material binden.

Die Erfinder stellen erstmals Nukleinsäuremoleküle bereit, die an komplexe native biologische Strukturen bzw. Endothelzellen binden. Die von Morris et al. bereitgestellten Sequenzmotive, die Bestandteile der dort verwendeten Nukleinsäuremoleküle bzw. Aptamere sind, binden hingegen an künstlich erzeugte Vesikel aus Erythrozytenzellmembranen. Die Integrität derart erzeugter Membranen entspricht nicht zwangsläufig der von physiologischen Erythrozytenzellmembranen. Ungeeignet ist hier auch die Wahl von Erythrozytenbestandteilen als Modell für eine komplexe biologische Struktur. Erythrozyten zeichnen sich durch einen hohen Differenzierungsgrad aus. Sie unterscheiden sich in einer Vielzahl von Eigenschaften von anderen typischen Zellen eines Organismus: keine Proteinsynthese, keine Einbindung in ein Zellverband, hohe Spezialisierung hinsichtlich Sauerstofftransport etc.

Die Erfinder haben zur Selektion ihrer spezifischen Nukleinsäuremoleküle als biologisches Material native Zellen einer Endothelzellinie verwendet. Dies hat den Vorteil, daß diese Nukleinsäuremoleküle auch *in vivo* zuverlässig eingesetzt werden können.

Die Erfinder haben zur Anreicherung funktioneller Nukleinsäuremoleküle ein Verfahren verwendet, mit den Schritten:
a) Inkubation einer Bibliothek von unterschiedlichen Nukleinsäuremolekülen mit einer Zielstruktur;
b) Selektion und Anreicherung der funktionellen Nukleinsäuremoleküle;
c) Isolierung der selektierten Nukleinsäuremoleküle, wobei zur Kontrolle der Anreicherung eine Fluoreszenzmarkierung der selektierten Nukleinsäuremoleküle verwendet wird.

Die Erfinder haben nämlich erkannt, daß zum Auffinden von spezifischen Nukleinsäuremolekülen bzw. Aptameren die Anreicherung der selektierten Nukleinsäuremoleküle nicht wie üblich durch Radioaktivmarkierung erfolgen muß, sondern daß hier eine Fluoreszenzmarkierung in Kombination mit geeigneten Detektionsverfahren verwendet werden kann. Es war nicht zu erwarten, daß durch solch eine Fluoreszenzmarkierung, die vorzugsweise am 5'-oder 3'-Ende der Sequenz erfolgt, die spezifischen Bindeeigenschaften der funktionellen Nukleinsäuremoleküle erhalten bleiben können. In den bis dato durchgeführten bekannten Verfahren wurde die Anreicherung funktioneller Nukleinsäuremoleküle in den Pools der fortschreitenden Selektionsrunden ausschließlich durch Szintillationsmessung der während der Anreicherung eingebauten radioaktiven dNTPs kontrolliert.

Die mit radioaktiven Arbeiten verbundenen Konsequenzen sind allgemein bekannt: Einrichtung eines speziellen Isotopenlabors, problematische Abfallentsorgung, strenge Vorschriften zur Lieferung und Lagerung des radioaktiven Materials, gesundheitliche Risiken, hohe Kosten, etc. Diese Probleme lassen sich durch den Einsatz alternativer Detektionstechniken, wie der Fluoreszenzmarkierung, umgehen.

Das neue Anreicherungsverfahren hat sich sehr ökonomisch erwiesen. Es erlaubt nicht nur die gleichzeitige Selektion gegen multiple Targets, die in ihre natürliche Umgebung aus ganzen Endothelzellen eingebettet sind, durch repetitive Zyklen von FACS-Messungen. Vielmehr ist auch eine in situ-Evaluierung von individuellen, spezifisch bindenden Aptameren mit Fluoreszenzmarkierung durch FACS möglich.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachstehend anhand von Beispielen erläutert, aus denen sich weitere Merkmale und Vorteile ergeben.

Die beigefügten Abbildungen dienen der Beschreibung der Erfindung. Es zeigen:
- Fig. 1: eine schematische Darstellung der Sekundärstruktur eines spezifischen Nukleinsäuremoleküls bzw. Aptamers,
- Fig. 2+3: die Charakterisierung der beanspruchten Nukleinsäuremoleküle mittels Durchflußcytometrie,
- Fig. 4: die Anreicherung der spezifischen Nukleinsäuremoleküle bzw. Aptamere in den Pools der fortschreitenden Selektionsversuchen,
- Fig. 5: die Anfärbung von Ratten-Glioblastom-Cryostat-Gewebeschnitten mit dem Nukleinsäuremolekül mit der Nukleotidsequenz SEQ ID Nr. 9, und
- Fig. 6: Coomassie-Blau gefärbtes Polyacrylamidgel zur Analyse des durch das Nukleinsäuremolekül mit der Nukleotidsequenz SEQ ID Nr. 9 erkannten Targets.

### Beispiel 1: Endothelzellen als biologische Zielstruktur

Als Zielstruktur für die Selektion der spezifischen Aptamere wird die Zellinie YPEN-1 (CRL-2222, American Type Culture Collection, Manassas, USA), eine endotheliale Prostatazellinie der Ratte, verwendet. Die Zellen werden durch Abschaben der subkonfluenten Endothelzellschicht geerntet. Die Konzentration der Zellen wird durch Zählen der Anzahl intakter Zellen nach Anfärbung mit Trypanblau in einer Neubauer-Zählkammer geschätzt.

### Beispiel 2: Start-(ssDNA-)Bibliothek und Primer

Die synthetische Start-Bibliothek (MWG Biotech AG, Ebersberg, Deutschland), aus der die spezifischen Nukleinsäuremoleküle bzw. Aptamere selektioniert werden, besteht aus näherungsweise 96mer Polynukleotiden, wobei um die 60 zufallssequenzierte Basen (60N) von jeweils 18 Nukleotiden definierter Sequenz, den Primerhybridisierungsstellen, flankiert werden (5'-ATA CCA GCT TAT TCA ATT-|60N|-AGA TAG TAA GTG CAA TCT-3'). Zur Amplifizierung spezifischer Nukleinsäuremoleküle mittels der unten aufgeführten PCR werden FITC-markierte und Biotin-markierte, HPLCgereinigte Primer (5'-FITC-18C-ATA CCA GCT TAT TCA ATT-3' und 5'-BBB-AGA TTG CAC TTA CTA TCT-3') verwendet, wobei 18C für einen 18-Carbonethylenglycol-Spacer, "FITC" und "B" für ein FITC-Molekül bzw. Biotin-Molekül steht), die mittels FestphasenSynthese synthetisiert werden (Operon Technologies, Inc., Alamenda USA).

### Beispiel 3: Gewinnung von endothelzellspezifischen Nukleinsäuremolekülen bzw. Aptameren

Zur Gewinnung der spezifischen Nukleinsäuremoleküle ist eine Selektion über mehrere Runden notwendig.

Die folgenden Schritte werden unter lichtgeschützten Bedingungen durchgeführt.

### Erste Runde

Ca. 1,7 nmol der DNA aus der Startbibliothek aus Beispiel 2 (1 x 10¹⁵ Sequenzen) werden in Selektionspuffer (50 mM Tris-HCl (pH 7,4), 5 mM KCl, 100 mM NaCl, 1 mM MgCl₂, 0,1 % NaN₃) gelöst (1 ml in der ersten Runde, 200 µl in den folgenden Runden). Die DNA wird denaturiert (80°C, 10 min) und bei 0°C für 10 min renaturiert. Um eine unspezifische Anlagerung der Polynukleotide an Zelloberflächen und Reaktionsgefäßwände zu verringern, wird jeweils ein 5-molarer Überschuß an tRNA (Gibco, Karlsruhe, Deutschland) und Rinderserumalbumin (BSA) zugegeben. Die DNA-Lösung wird mit 3 x 10⁷ N9-Mikrogliazellen für 30 min bei 37°C / 5 % CO₂ präinkubiert, um DNA-Moleküle mit hoher Affinität zu irrelevanten Oberflächenproteinen zu entfernen.

Die Mikrogliazellen werden abzentrifugiert und der Überstand wird mit 10⁶ YPEN-Endothelzellen bei 37°C/5 % CO₂ inkubiert. Nach dreimaligem Waschen durch Zentrifugation mit jeweils 1 ml Selektionspuffer (plus 0,2 % BSA), werden die endothelzellgebundenen Nukleinsäuremoleküle als Template benutzt, um Aptamere mittels PCR zu amplifizieren (Taq-Polymerase und dNTPs stammen von Promega, Mannheim, Deutschland).

Die PCR erfolgt unter Bedingungen nach Crameri und Stemmer, "10(20)-fold aptamer library amplification without gel purification", Nucl. Acids Res. 1993; 21(18): 4410. Als Primer werden die oben beschriebenen FITC- und Biotin-markierten Primer verwendet.

### Gewinnung FITC-markierter Einzelstränge

Zur fluoreszenzzytometrischen Analyse wird FITC-markierte ssDNA wie folgt dargestellt: FITC-konjugierte ssDNA wird vom biotinylierten Strang durch Isolierung der PCR-dsDNA-Produkte mit der entsprechenden Menge an magnetischen Streptavidinbeads (Dynabeads M-280 Streptavidin; Dynal Hamburg, Deutschland) in einem Magnetständer, gefolgt von alkalischer Denaturierung gemäß den Anweisungen des Herstellers, gereinigt und in einem Endvolumen von 200 µl Selektionspuffer aufgenommen. Die vorliegenden ssDNA-Lösung dient als Pool für die jeweils folgende Selektionsrunde.

### Zweite und dritte Selektionsrunde

Der neue FITC-ssDNA-Pool wird erneut gegen Mikroglia in Polystyren-Reaktionsgefäßen präinkubiert. Die Selektion erfolgt (ebenfalls in Polystyren-Reaktionsgefäßen) gegen 1,5 x 10⁵ Endothelzellen. Nach 30 min Inkubation bei 37°C werden die Zellen durch dreimaliges Waschen mit 1 ml Selektionspuffer plus 0,2 % BSA von weniger spezifisch bindender DNA sowie von Sequenzen, deren 5'-Modifikation zu Bindungsverlust führt, befreit. Die Fluoreszenz der Endothel-Nukleinsäuremolekül-Komplexe wird am Durchflußzytometer (FACS) gemessen. Die Amplifizierung der ssDNA erfolgt wie in der vorangegangenen Runde.

### Vierte bis achte Selektionsrunde

Nach erneuter Präinkubation gegen Mikroglia (wie bereits beschrieben) erfolgt die Selektion gegen 1 x 10⁵ Endothelzellen.

Das Ausscheiden von Separationsmatrix-bindenden Sequenzen wird durch Austausch des Polystyren-Reaktionsgefäßes gegen ein Polypropylen-Reaktionsgefäß gewährleistet. Der 30-minütigen Inkubation mit dem Waschen der Zellen (wie in den vorangehenden Runden) folgt wiederum die Bestimmung der Fluoreszenzintensität am Durchflußzytometer (Zählen von 5.000 Zellen pro Selektionsrunde). Zellgebundene Nukleinsäuremoleküle werden, wie bereits beschrieben, PCR-amplifiziert.

### Gewinnung einzelner Klone aus dem Pool der Runde 8

Die spezifischen Nukleinsäuremoleküle aus der siebten Selektionsrunde (Runde 8. Pool) werden mit unmodifizierten Primern PCR-amplifiziert und in E. coli kloniert (TA-Cloning; Invitrogen Groningen, Niederlande). Die Plasmide der einzelnen Klone werden durch alkalische Lyse isoliert und das Insert (PCR-Produkt) wie in den Selektionsrunden mit den modifizierten Primern PCR-amplifiziert. Die Einzelstranggewinnung erfolgt wie bereits beschrieben.

Mit Hilfe dieser FITC-Aptamer-Lösungen erfolgt die Charakterisierung der Bindungsspezifität individueller Sequenzen mittels Durchflußzytometrie (FACS) und histologischen Bindestudien.

### FACS-Messung

Sowohl die spezifische Bindung der Aptamere in den fortschreitenden Selektionsrunden als auch die spezifische Bindung der einzelnen Klone werden am FACS (Fluorescence-Activated Cell Sorting) gemessen.

Zellen (als signalgebende Ereignisse) emittieren proportional zu der Intensität ihrer Fluoreszenzmarkierung elektromagnetische Strahlung. Diese Strahlung wird detektiert und quantifiziert. Sie erlaubt somit Rückschluß auf die Menge an zellgebundenen, fluoreszenzmarkierten Liganden. In einem Histogramm wird die Fluoreszenzintensität gegen die Anzahl der signalgebenden Ereignisse aufgetragen. Eine starke Ablenkung in Richtung zunehmender Fluoreszenz ist somit ein Maß für eine hohe Affinität der spezifischen Nukleinsäuremoleküle bzw. Aptamere zu dem verwendeten biologischen Targetmaterial bzw. zu den hier verwendeten Endothelzellen.
(a) FACS-Analyse von FITC-gekoppelten Aptameren in den fortschreitenden Selektionsrunden
   Das spezifische Binden von FITC-gekoppeltem Aptamer wird in der zweiten und in den folgenden Selektionsrunden gemessen. Nach der Inkubation der Nukleinsäuremoleküle bzw. Aptamere mit den Endothelzellen (wie oben beschrieben) werden die Endothelzellen zweimal mit Selektionspuffer (plus 0,2 % BSA) gewaschen und die Zellfluoreszenz am FACS gemessen.
(b) FACS-Analyse der spezifischen Bindung der einzelnen FITC-gekoppelten Klone
   Dazu werden 5 x 10⁴ YPEN-Endothelzellen mit Selektionspuffer (plus 1 µg/µl tRNA; 20 min; 0°C) präinkubiert und mit der oben beschriebenen FITC-ssDNA-Lösung inkubiert (plus 1 µg/µl tRNA; 37°C / 5 % CO₂ / 30 min). Die Zellen werden zweimal mit Selektionspuffer (plus 0,2 % BSA) gewaschen und die Zell-Fluoreszenz am FACS gemessen.

### Histologische Bindestudien an Gewebeschnitten

Die Bindung der gewonnenen individuellen FITC-modifizierten Nukleinsäuremoleküle bzw. Aptamere wird durch histologische Bindestudien an Ratten-Glioblastom-Cryostat-Gewebeschnitten getestet. Die Gewebeschnitte werden mit 1 µg/µl tRNA in Selektionspuffer präinkubiert (20 min, 4°C). Die präinkubierten Gewebeschnitte werden mit der oben beschriebenen FITC-Aptamer-Lösung (plus 1 µg/µl tRNA) bedeckt und bei Raumtemperatur 40 min inkubiert. Die Gewebeschnitte werden zweimal mit Selektionspuffer gewaschen und die Immunfluoreszenz mittels Fluoreszenzmikroskopie bestimmt.

Die gefundenen hochspezifischen Nukleinsäuremoleküle bzw. Aptamere zeichnen sich dadurch aus, daß diese selektiv die neu formierten Mikrogefäße des Glioblastomgewebes anfärben und minimale Kreuzreaktion mit anderen nicht-endothelialen Strukturen zeigen. Die Nukleinsäuremoleküle sind somit besonders geeignet, als histologische Sonden zur Anfärbung von Endothelzellen eingesetzt zu werden.

### Beispiel 4: Spezifische Nukleinsäuremoleküle bzw. Aptamere gegen (endothelzell-)spezifisches Antigen

Die folgenden. Nukleotidsequenzen wurden mit Hilfe des vorstehenden Verfahrens gewonnen und sind Bestandteil der vorliegenden Erfindung. Die Primerhybridisierungsstellen liegen 5'-und 3'-wärts der eigentlichen Aptamersequenzen (fett gedruckt).

### Nukleinsäuremolekül 1 (Primärstruktur)

### Nukleinsäuremolekül 2 (Primärstruktur)

### Nukleinsäuremolekül 3 (Primärstruktur)

### Nukleinsäuremolekül 4 (Primärstruktur)

### Nukleinsäuremolekül 5 (Primärstruktur)

### Nukleinsäuremolekül 6 (Primärstruktur)

### Nukleinsäuremolekül 7 (Primärstruktur)

### Nukleinsäuremolekül 8 (Primärstruktur)

### Nukleinsäuremolekül 9 (Primärstruktur)

### Nukleinsäuremolekül 10 (Primärstruktur)

### Nukleinsäuremolekül 11 (Primärstruktur)

### Nukleinsäuremolekül 12 (Primärstruktur)

### Nukleinsäuremolekül 13 (Primärstruktur)

### Nukleinsäuremolekül 14 (Primärstruktur)

### Nukleinsäuremolekül 15 (Primärstruktur)

### Nukleinsäuremolekül 16 (Primärstruktur)

### Nukleinsäuremolekül 17 (Primärstruktur)

### Nukleinsäuremolekül 18 (Primärstruktur)

### Nukleinsäuremolekül 19 (Primärstruktur)

### Nukleinsäuremolekül 20 (Primärstruktur)

### Nukleinsäuremolekül 21 (Primärstruktur)

### Beispiel 5: Modell der Struktur eines spezifischen Nukleinsäuremoleküls bzw. Aptamers

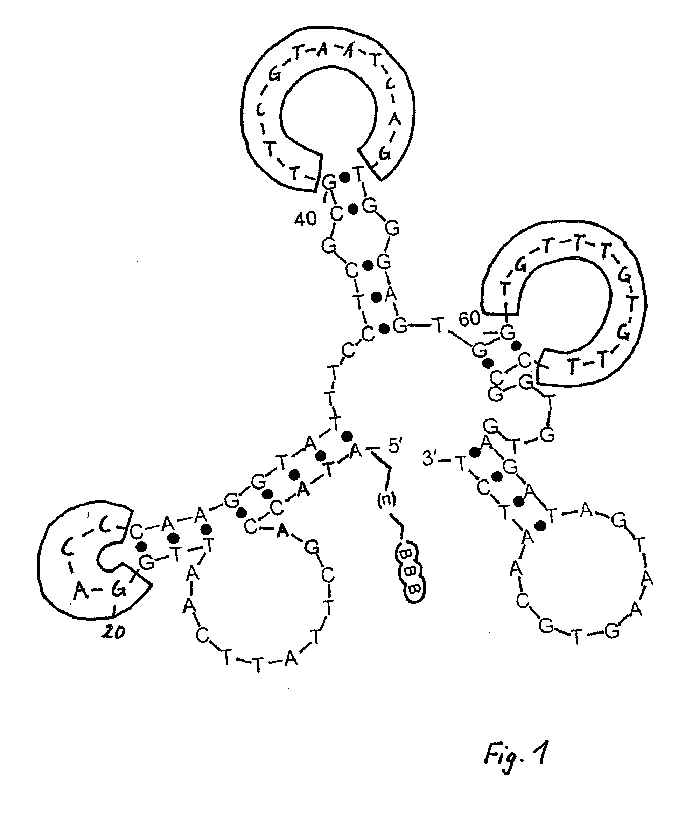

Fig. 1 stellt die Sekundärstruktur einer hypothetischen spezifischen Nukleotidsequenz bzw. Aptamersequenz dar. Die aktiven Bindestellen, die sich innerhalb verschiedener sogenannter "hairpin loops" bzw. "bulgs" befinden, sind hervorgehoben. Das dargestellte Nukleinsäuremolekül ist am 5'-Ende über einen Linker-Abschnitt (n) biotin-markiert (BBB). Ausgefüllte Kreise symbolisieren Basenpaarungen. Positionsangaben kennzeichnen die Lage der Nukleotide im Molekül.

### Beispiel 6: Histogramme der beanspruchten Nukleinsäuremoleküle

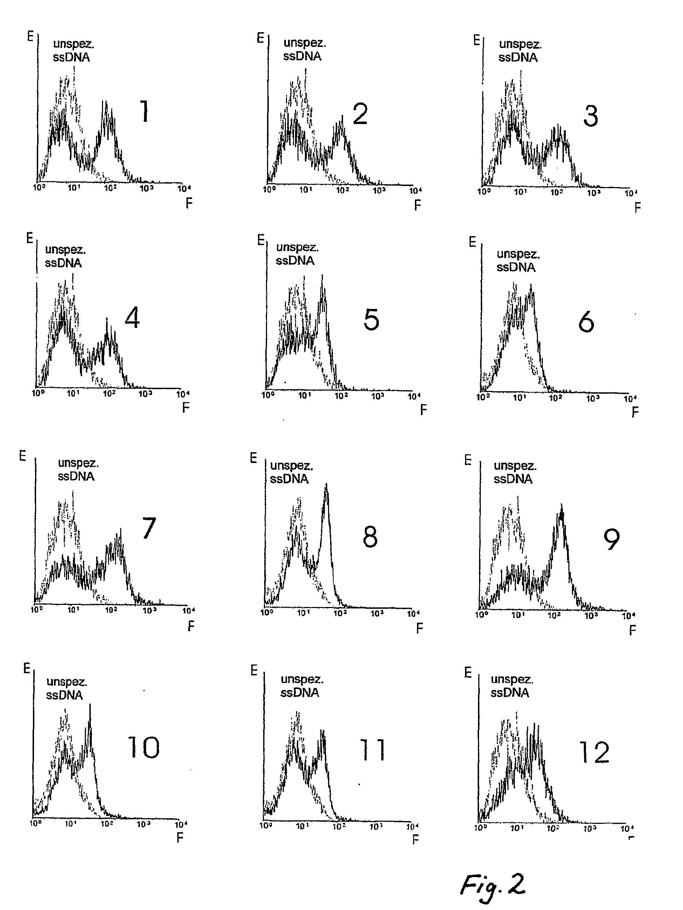

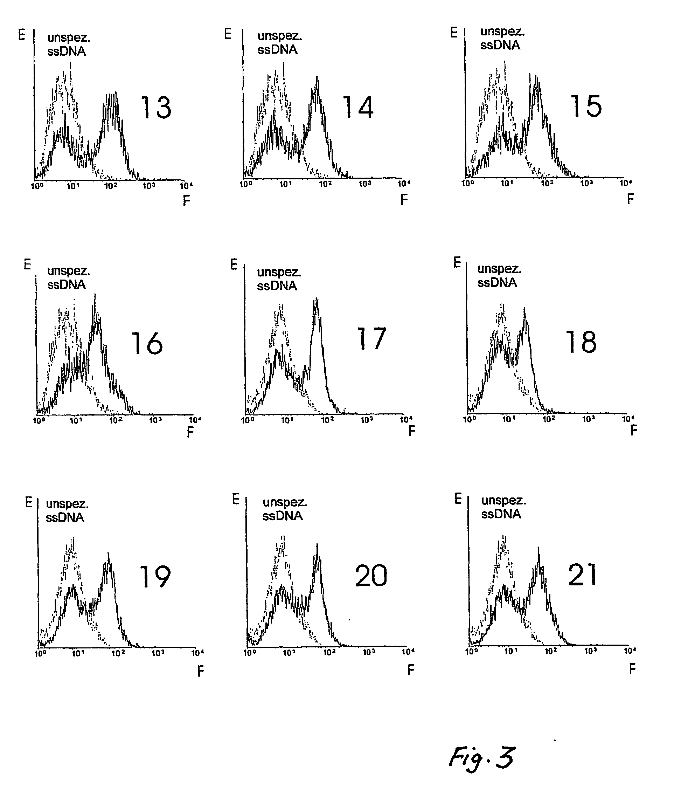

In Fig. 2 und 3 sind die FACS-Histogramme der gefundenen Nukleinsäuremoleküle dargestellt. Auf der horizontalen Achse ist die Fluoreszenzintensität (F) in willkürlichen logorithmischen Einheiten aufgetragen, die vertikale Achse zeigt die Anzahl der signalgebenden Ereignisse (E), aufgetragen in willkürlichen linearen Einheiten. Die Histogramme demonstrieren die Spezifität der Bindung der Nukleinsäuremoleküle SEQ ID Nr. 1 bis 21 an die Zielstruktur. Die Verschiebung der Histogramm-Kurve der Nukleinsäuremoleküle (SEQ ID Nr. 1 bis 21, fett gedruckt) in Richtung zunehmender stärkerer Fluoreszenz zeigt die hohe Affinität dieser Moleküle zur Zielstruktur im Vergleich zur Referenz. Als Referenz ist jeweils das Fluoreszenzsignal bei Verwendung von unspezifischer gleichartig FITC-gekoppelter Einzelstrang-DNA (unspez. ssDNA, grau gedruckt) dargestellt. Alle 21 Nukleinsäuremoleküle sind somit durch eine hohe Affinität zu den verwendeten Endothelzellen charakterisiert.

### Beispiel 7: Histogramme der Nukleinsäuremoleküle in den komplexen Pools der Selektionsrunden

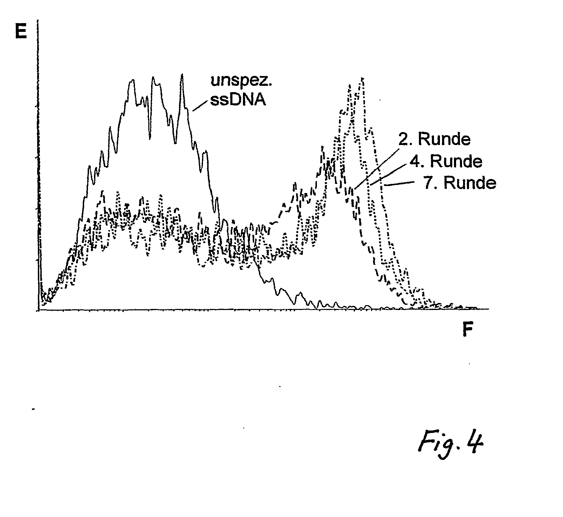

In Fig. 4 sind die FACS-Histogramme der Aptamer-Pools aus der zweiten (----), vierten (.....) und siebten (-·-·-·-·-) Selektionsrunde überlagert dargestellt. Die Verschiebung der Histogramm-Kurve der dargestellten Selektionsrunden in Richtung zunehmender stärkerer Fluoreszenz verbildlicht die Zunahme spezifisch bindender Nukleinsäuremoleküle (Aptamere) in den Pools der voranschreitenden Selektionsrunden. Als Referenz ist das Fluoreszenzsignal bei Verwendung von unspezifischer gleichartig FITC-gekoppelter Einzelstrang-DNA (unspez. ssDNA, grau gedruckt) dargestellt.

### Beispiel 8: Charakterisierung des Nukleinsäuremoleküls mit der Nukleotidsequenz SEQ ID Nr. 9 als tumor- und 'pigpen'-spezifisches Aptamer

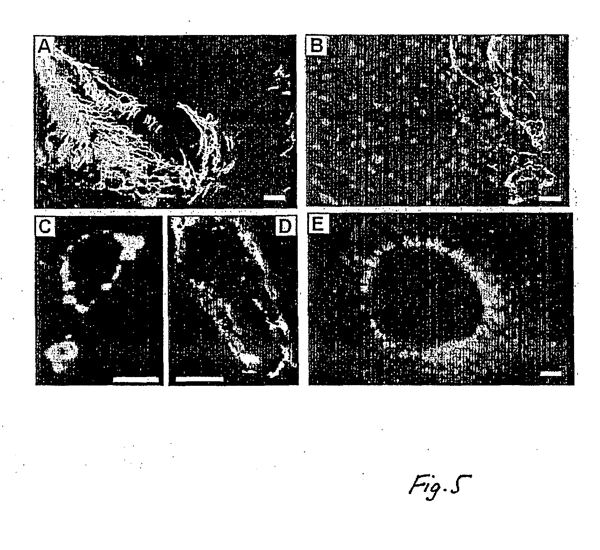

In von den Erfindern durchgeführten histologischen Bindestudien, die am Ende des Ausführungsbeispiels 3 beschrieben sind, hat sich herausgestellt, daß das Nukleinsäuremolekül mit der Nukleotidsequenz SEQ ID Nr. 9 auf Gewebeschnitten von Ratten-Glioblastomen von sämtlichen neuen Nukleinsäuremolekülen das stärkste Fluoreszenzsignal, und zwar spezifisch in Bereichen starken Tumorwachstums, zeigt. Dies ist in der Fig. 5 dargestellt. Hier ist in der Teilabbildung A die selektive Anfärbung von Mikrogefäßen dargestellt, die in das komplexe Gewebe des Glioblastoms eingebettet sind. Teilabbildung B zeigt die Gegenfärbung der Zellkerne mit DAPI, wodurch das selektive Markieren der Mikrogefäße mit dem genannten Aptamer in der zellreichen Tumorregion (die fädigen hellen Strukturen auf der rechten Seite der Aufnahme stellen die angefärbten Mikrogefäße dar), nicht aber von den Gefäßen in den peritumoralen Bereichen (linke Seite der Aufnahme) ersichtlich wird. Teilabbildung C zeigt eine Doppelfärbung mit dem käuflichen endothelialen monoklonalen Antikörper CD-31 (Dako, Hamburg, Deutschland), was zu einer Überlagerung beider Fluoreszenzsignale führt, wodurch die durch das in Rede stehende Aptamer markierten Zellen als Endothelzellen bestimmt werden. Teilabbildung D zeigt eine Anfärbung mit dem monoklonalen Antikörper CD-31 und einem unspezifischen sequenzrandomisierten Aptamer, wobei keinerlei Doppelfärbung von Endothelzellen oder gefäßassoziierten Strukturen bzw. keine Signalüberlagerung zu erkennen ist. Teilabbildung E zeigt eine Anfärbung einer Endothelzellkultur nach dem Verwunden einer konfluenten Zellschicht mit einer Pipettenspitze. Hier zeigt das in Rede stehende Aptamer eine signifikant erhöhte Bindung an die Endothelzellen in dem subkonfluenten verwundeten kreisförmigen Bereich. Der Skalenstrich entspricht 50 µm.

Dieses Experiment zeigt, daß das Aptamer mit der Nukleotidsequenz SEQ ID Nr. 9 spezifisch die komplexe Architektur der pathologischen Mikrogefäße anfärbt. Die Gegenfärbung der Zellkerne mit DAPI unterstreicht, daß selektiv nur Mikrogefäße innerhalb der zellreichen Tumorregion, nicht aber Gefäße in peritumoralem Gewebe erkannt werden. Die Erfinder konnten in weiteren, hier nicht gezeigten Experimenten, bei denen normales Rattengehirn mit dem Aptamer angefärbt wurde, in fluoreszenzmikroskopischen Aufnahmen keinerlei Anfärbung von Endothelzellen oder gefäßassoziierten Strukturen feststellen. Die Anfärbung von Tumorgewebeschnitten mit Maus-IgG, die gegen Endothelzellen gerichtet sind, d.h. bspw. mit dem obengenannten CD-31-Antikörper und der anschließenden Markierung mit dem in Rede stehenden Aptamer bestätigt das Aptamertarget als Endothelzellstruktur. Die Anfärbung von verletztem Endothelzellgewebe zeigt eine deutliche Hochregulierung des molekularen Targets des Aptamers im Bereich der Läsion, d.h. in einer Region, die durch verstärkte Proliferation - im Vergleich zu den Bereichen des konfluenten Monolayers - gekennzeichnet ist.

Um das Target identifizieren zu können, das von dem Aptamer mit der Nukleotidsequenz SEQ ID Nr. 9 erkannt wird, wird wie folgt vorgegangen: 1 mg (100 µl) von magnetischen Streptavidin-Beats werden mit 200 pmol dreifach biotinyliertem (trB) Aptamer durch Inkubation in 1 ml Selektionspuffer (30 Minuten, Raumtemperatur) gekoppelt (MWG-Biotech AG, Ebersberg, Deutschland). Als Kontrolle werden 100 µl magnetische Beats mit 200 pmol unspezifischer FITC-ssDNA gekoppelt (trB-96-nt). In Gegenwart von Proteaseinhibitoren werden 1,5 x 10⁸ YPEN-1 Endothelzellen lysiert, wie beschrieben in Klocker et al., Journal of Neuroscience 19, 8517-8527 (1999). Nach der Zentrifugation wird das Proteinpellet in 400 µl Selektionspuffer resuspendiert, ultrasonifiziert (0°C, 20 Sekunden) und in Selektionspuffer (Gesamtvolumen 1,5 ml, 0°C, 15 Minuten) mit den aptamerbedeckten magnetischen Beats unter Hinzufügung des hundertfachen Überschusses von tRNA (20 nmol) als einem unspezifischen Kompetitor inkubiert. Der Komplex aus Protein und den gekoppelten Aptameren wird in einem Magnetständer rausgelöst und fünf Mal gewaschen (1. Waschvorgang: 1 ml Selektionspuffer mit 150 mM NaCl; 2. bis 5. Waschvorgang: 200 µl Selektionspuffer mit 100 mM NaCl mit 2 nmol tRNA). Das Protein wird von den aptamerbeschichteten Beats durch Inkubation in 30 µl 1 M NaCl entfernt (0°C, 30 Minuten) und durch PAGE und anschließendem Anfärben mit Comassie-Blau analysiert.

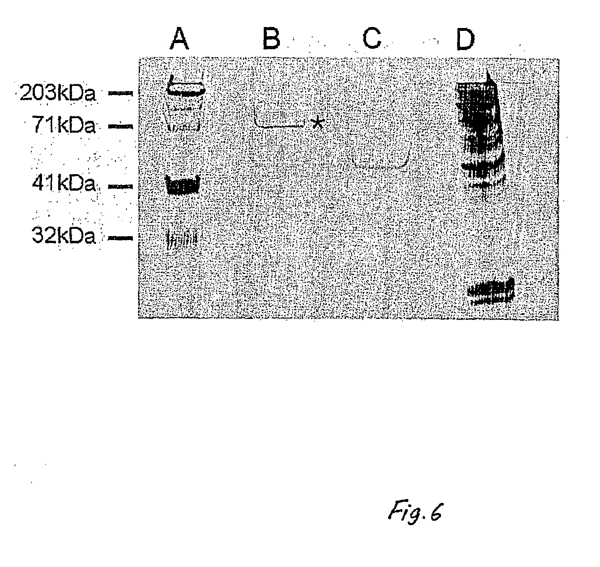

Ein solches Experiment ist in der Fig. 6 dargestellt. In Spur A ist der molekulare Marker aufgetragen, in Spur B das Target, das mit dem Aptamer mit der Nukleotidsequenz SEQ ID Nr. 9 isoliert wurde, in der Spur C als Kontolle das Isolat, das mit unspezifischer ssDNA (96-nt) gewonnen wurde und in Spur D sind unbehandelte Endothelzellen aufgetragen. Die Targetbande, die mittels des in Rede stehenden Aptamers isoliert wurde, ist durch einen Stern gekennzeichnet und weist ein Molekulargewicht von etwa 67 kDa auf.

### Dieses Targetprotein wird wie folgt identifiziert:

Das entsprechende Gelstück wird ausgeschnitten und ein tryptischer Verdau wird durchgeführt, wie dies z.B. beschrieben ist in Shevchenko et al., Anal. Chem. 68, 850-858 (1996). Dabei werden, im Anschluß kurz beschriebene, Modifizierungen vorgenommen. Die ausgeschnittene Proteinbande wird vollständig entfärbt und für drei Stunden mit Schweinetrypsin (sequencing grade, modifiziert, Promega, Mannheim, Deutschland) bei einer Konzentration von 67 ng pro µl in 25 mM Ammoniumbicarbonat (pH 8,1 bei 37°C) verdaut. Vor dem Peptidmapping und der Sequenzierung der tryptischen Fragmente durch Tandemmassenspektrometrie wird das Peptidgemisch aus dem Gel durch Behandlung mit einer Lösung aus 50 % Trifluoressigsäure/50 % Wasser, gefolgt von einer Behandlung mit 50 % Trifluoressigsäure/50 % Acetonitril, extrahiert. Die Extrakte werden vakuumgetrocknet. Die getrockneten Peptide werden in 0,1 % Trifluoressigsäure resuspendiert und unter Verwendung einer Ready-to-go-Pipettenspitze mit C18-spherical-silica-reverse-Phase-Material (ZipTip_{C18}TM, Millipore) gereinigt. Die Peptide werden mit 10 µl 50 % Methanol/1 % Ameisensäure eluiert und die Sequenzierung wird mittels Nano-Elektrospray-Tandem-Massenspektrometrie (Q-Tof, Micromass, Manchester, England) durchgeführt.

Die Erfinder haben das von dem Aptamer mit der Nukleotidsequenz SEQ ID Nr. 9 erkannte Target einer solchen Analyse unterzogen. Dabei wurden drei tryptische Peptidfragmente des Proteins durch Massenspektrometrie bzw. Tandem-Massenspektrometrie untersucht bzw. sequenziert. Dabei stellte sich heraus, daß das von dem in Rede stehenden Aptamer erkannte Target ein Homolog des 'pigpen'-Proteins der Maus ist.

## Patentansprüche

1. Nukleinsäuremolekül mit der Nukleotidsequenz SEQ ID Nr. 9 aus dem beiliegenden Sequenzprotokoll.

2. Nukleinsäuremolekül nach Anspruch 1, **dadurch gekennzeichnet, daß** es an ein Therapeutikum und/oder Diagnostikum gekoppelt ist.

3. Verwendung eines Nukleinsäuremoleküls nach Anspruch 1 oder 2 für histologische Untersuchungen von Gewebeschnitten.

4. Verwendung eines Nukleinsäuremoleküls, das die Nukleotidsequenz SEQ ID Nr. 9 aus dem beiliegenden Sequenzprotokoll enthält, zum in-vitro Nachweis einer Tumorerkrankung endothelialen Ursprungs in der Ratte.

5. Verwendung nach Anspruch 4, **dadurch gekennzeichnet, daß** der Nachweis über das Protein 'pigpen' erfolgt.

6. In-vitro Verfahren, bei dem ein Nukleinsäuremolekül selektiv an Endothelzellen bindet oder diese markiert, **dadurch gekennzeichnet, daß** das Nukleinsäuremolekül die Nukleotidsequenz SEQ ID Nr. 9 aus dem beiliegenden Sequenzprotokoll enthält.

7. Verfahren nach Anspruch 6, **dadurch gekennzeichnet, daß** das Nukleinsäuremolekül ein Nukleinsäuremolekül mit der Nukleotidsequenz SEQ ID Nr. 9 aus dem beiliegenden Sequenzprotokoll ist.

8. In-vitro Verfahren zum Nachweisen einer Tumorerkrankung endothelialen Ursprungs in der Ratte mit dem Schritt:
a) Nachweis eines Tumormarkers in bereitgestelltem biologischem Material
**dadurch gekennzeichnet, daß**
der Nachweis mit einem Nukleinsäuremolekül erfolgt, das die Nukleotidsequenz SEQ ID Nr. 9 aus dem beiliegenden Sequenzprotokoll enthält.

9. Verfahren nach Anspruch 10, **dadurch gekennzeichnet, daß** der Nachweis über das Protein 'pigpen' erfolgt.

10. Verfahren nach Anspruch 8 oder 9, **dadurch gekennzeichnet, daß** das Nukleinsäuremolekül an ein Therapeutikum gekoppelt ist.

11. Verfahren nach einem der Anspruche 8 bis 10, **dadurch gekennzeichnet, daß** das Nukleinsäuremolekül an ein Diagnostikum gekoppelt ist.

12. Verfahren nach Anspruch 11, **dadurch gekennzeichnet, daß** das Diagnostikum aus der Gruppe ausgewählt ist, die enthält: FITC, Biotin, Digoxigenin und deren Abkömmlinge, sowie weitere fluoreszierende Substanzen, enzymatische Marker, Infrarot-Marker und Chelatbildner.

13. Verwendung eines Nukleinsäuremoleküls, das die Nukleotidsequenz SEQ ID Nr. 9 aus dem beiliegenden Sequenzprotokoll enthält, in dem Verfahren nach einem der Ansprüche 6 bis 12, **dadurch gekennzeichnet, daß** das Nukleinsäuremolekül selektiv an natives komplexes biologisches Material bindet.

14. In-vitro Verfahren zum Detektieren oder Kontaktieren von entzündlichen Endothelzell-spezifischen Läsionen, **dadurch gekennzeichnet, daß** die Nukleinsäuremoleküle aus einem der Ansprüche 1 oder 2 mit biologischem Material kontaktiert werden.

15. Verfahren nach Anspruch 14, **dadurch gekennzeichnet, daß** an das Nukleinsäuremolekül ein Therapeutikum gekoppelt ist.

## Claims

1. A nucleic acid molecule comprising the nucleotide sequence SEQ ID No. 9 from the enclosed sequence listing.

2. The nucleic acid molecule of claim 1, **characterized in that** it is coupled to a therapeutic and/or diagnostic agent.

3. Use of a nucleic acid molecule of claim 1 or 2 for histologic analyses of tissue sections.

4. Use of a nucleic acid molecule which contains the nucleotide sequence SEQ ID No. 9 from the enclosed sequence listing for the detection *in vitro* of a tumor disease of endothelial origin in the rat.

5. Use of claim 4, **characterized in that** the detection takes place via the protein 'pigpen'.

6. *In-vitro*-method, wherein a nucleic acid molecule selectively binds to or labels endothelial cells, **characterized in that** the nucleic acid molecule contains the nucleotide sequence SEQ ID No. 9 from the enclosed sequence listing.

7. Method of claim 6, **characterized in that** the nucleic acid molecule is a nucleic acid molecule comprising the nucleotide sequence SEQ ID No. 9 from the enclosed sequence listing.

8. *In*-*vitro*-method for the detection of a tumor disease of endothelial origin in the rat, comprising the steps:
a) detection of a tumor marker in provided biological material,
**characterized in that**
the detection takes place with a nucleic acid molecule which contains the nucleotide sequence SEQ ID No. 9 from the enclosed sequence listing.

9. Method of claim 10, **characterized in that** the detection takes place via the protein 'pigpen'.

10. Method of claim 8 or 9, **characterized in that** the nucleic acid molecule is coupled to a therapeutic agent.

11. Method of any of claims 8 to 10, **characterized in that** the nucleic acid molecule is coupled to a diagnostic agent.

12. Method of claim 11, **characterized in that** the diagnostic agent is selected from the group which contains: FITC, Biotin, Digoxigenin and its derivatives, as well as further fluorescent substances, enzymatic markers, infrared markers and chelating agents.

13. Use of a nucleic acid molecule which contains the nucleotide sequence SEQ ID No. 9 from the enclosed sequence listing, in a method of any of claims 6 to 12, **characterized in that** the nucleic acid molecule selectively binds to native complex biological material.

14. *In-vitro*-method for the detection or contacting of inflammatory endothelial cell specific lesions, **characterized in that** the nucleic acid molecules of claims 1 or 2 are contacted with biological material.

15. Method of claim 14, **characterized in that** the nucleic acid molecule is coupled to a therapeutic agent.

## Revendications

1. Molécule d'acide nucléique ayant la séquence nucléotidique SEQ ID n°. 9 du listage de séquences joint.

2. Molécule d'acide nucléique selon la revendication 1 **caractérisée en ce qu'**elle est couplée à un agent thérapeutique et/ou diagnostique.

3. Utilisation d'une molécule d'acide nucléique selon la revendication 1 ou 2 pour des études histologiques de coupes tissulaires.

4. Utilisation d'une molécule d'acide nucléique, qui contient la séquence nucléotidique SEQ ID n°. 9 du listage de séquences joint, pour la mise en évidence in vitro d'une maladie tumorale d'origine endothéliale chez le rat.

5. Utilisation selon la revendication 4 **caractérisée en ce que** la mise en évidence a lieu par le biais de la protéine « pigpen ».

6. Procédé in vitro dans lequel une molécule d'acide nucléique se lie sélectivement aux cellules endothéliales ou les marque, **caractérisé en ce que** la molécule d'acide nucléique contient la séquence nucléotidique SEQ ID n°. 9 du listage de séquences joint.

7. Procédé selon la revendication 6, **caractérisé en ce que** la molécule d'acide nucléique est une molécule d'acide nucléique ayant la séquence nucléotidique SEQ ID n°. 9 du listage de séquences joint.

8. Procédé in vitro pour la mise en évidence d'une maladie tumorale d'origine endothéliale chez le rat comportant l'étape :
a) mise en évidence d'un marqueur tumoral dans une matière biologique préparée,
**caractérisé en ce que**
la mise en évidence a lieu avec une molécule d'acide nucléique qui contient la séquence nucléotidique SEQ ID n°. 9 du listage de séquences joint.

9. Procédé selon la revendication 10 **caractérisé en ce que** la mise en évidence a lieu par le biais de la protéine « pigpen ».

10. Procédé selon la revendication 8 ou 9 **caractérisé en ce que** la molécule d'acide nucléique est couplée à un agent thérapeutique.

11. Procédé selon l'une des revendications 8 à 10 **caractérisé en ce que** la molécule d'acide nucléique est couplée à un agent diagnostique.

12. Procédé selon la revendication 11 **caractérisé en ce que** l'agent diagnostique est choisi dans le groupe qui contient : FITC, la biotine, la digoxigénine et ses dérivés, ainsi que d'autres substances fluorescentes, les marqueurs enzymatiques, les marqueurs infrarouges et les chélateurs.

13. Utilisation d'une molécule d'acide nucléique qui contient la séquence nucléotidique SEQ ID n°. 9 du listage de séquences joint dans le procédé selon l'une des revendications 6 à 12 **caractérisée en ce que** la molécule d'acide nucléique se lie sélectivement à une matière biologique complexe native.

14. Procédé in vitro pour la détection ou la mise en contact de lésions inflammatoires spécifiques des cellules endothéliales **caractérisé en ce que** les molécules d'acide nucléique de l'une des revendications 1 ou 2 sont mises en contact avec une matière biologique.

15. Procédé selon la revendication 14 **caractérisé en ce qu'**un agent thérapeutique est couplé à la molécule d'acide nucléique.


### SEQUENZPROTOKOLL

<110> Eberhard-Karls-Universität Tübingen UKT
<120> Zellbindende Nukleinsäuremoleküle (Aptamere)
<130> 5402P189WO
<140>
   <141>
<160> 21
<170> PatentIn Ver. 2.1
<210> 1
   <211> 96
   <212> DNA
   <213> Künstliche Sequenz
<220>
   <223> Beschreibung der künstlichen Sequenz: Nukleotidsequenz
<400> 1
   ataccagctt attcaattgg acccaaggta tttcctcgcg ttcgtaatca gtgggagtgg 60
   tgtttgtgtt ccggtgtgag atagtaagtg caatct                            96
<210> 2
   <211> 95
   <212> DNA
   <213> Künstliche Sequenz
<220>
   <223> Beschreibung der künstlichen Sequenz: Nukleotidsequenz
<400> 2
   ataccagctt attcaattct accagactgt ctttcaccct gcgccgttgt ggtctgttcg 60
   ttgttctagt tgttttcaga tagtaagtgc aatct 95
<210> 3
   <211> 96
   <212> DNA
   <213> Künstliche Sequenz
<220>
<223> Beschreibung der künstlichen Sequenz:
   Nukleotidsequenz
<400> 3
   ataccagctt attcaattgt cagcttttta gtgattttgg gttttttggt gtacgtccct 60
   gtaaathagt ttcagtcgag atagtaagtg caatct 96
<210> 4
   <211> 96
   <212> DNA
   <213> Künstliche Sequenz
<220>
<223> Beschreibung der künstlichen Sequenz:
   Nukleotidsequenz
<400> 4
   ataccagctt attcaattga gtgctgattc ccgtttctct ctggtatcga attgaggtcg 60
   tttgtgtgtg agttggctag atagtaagtg caatct 96
<210> 5
   <211> 96
   <212> DNA
   <213> Künstliche Sequenz
<220>
   <223> Beschreibung der künstlichen Sequenz: Nukleotidsequenz
<400> 5
   ataccagctt attcaattcc aacaccataa cctttctttg acctgacttt agccgtaatg 60
   tatttgggcc atccccttag atagtaagtg caatct 96
<210> 6
   <211> 137
   <212> DNA
   <213> Künstliche Sequenz
<220>
   <223> Beschreibung der künstlichen Sequenz: Nukleotidsequenz
<400> 6
   ataccagctt attcaattgg gtggagatag taggtgcaat ctataccagc ttattcaatt 60
   gccccgattt ggatgtaatt attgcgcgtg tatttttgat tgtataaagt gttgctacaa 120
   gatagtaagt gcaatct                                                137
<210> 7
   <211> 98
   <212> DNA
   <213> Künstliche Sequenz
<220>
   <223> Beschreibung der künstlichen Sequenz: Nukleotidsequenz
<400> 7
   ataccagctt attcaattag gcagatgaga agttaaggcg gtgctataga tggaccattt 60
   aggattttat ggttgggcgt agatagtaag tgcaatct                         98
<210> 8
   <211> 95
   <212> DNA
   <213> Künstliche Sequenz
<220>
   <223> Beschreibung der künstlichen Sequenz:
   Nukleotidsequenz
<400> 8
   ataccagctt attcaattcc cgcctttact tgggagatta tcaccgcggt atataaatac 60
   tgttcggagt tctgtgtaga tagtaagtgc aatct                            95
<210> 9
   <211> 93
   <212> DNA
   <213> Künstliche Sequenz
<220>
   <223> Beschreibung der künstlichen Sequenz: Nukleotidsequenz
<400> 9
   ataccagctt attcaattag gcggtgcatt gtggttggta gtatacatga ggtttggaga 60
   ctagtcgcaa gatatagata gtaagtgcaa tct 93
<210> 10
   <211> 97
   <212> DNA
   <213> Künstliche Sequenz
<220>
   <223> Beschreibung der künstlichen Sequenz: Nukleotidsequenz
<400> 10
   ataccagctt attcaattct gttggacatt caaaagacta gttcacgtcc gttgcccatt 60
   cttccctttg ttgactgcta gatagtaagt gcaatct 97
<210> 11
   <211> 96
   <212> DNA
   <213> Künstliche Sequenz
<220>
   <223> Beschreibung der künstlichen Sequenz: Nukleotidsequenz
<400> 11
   ataccagctt attcaattcg gctaggctcc attaagggtg acttatgggc caaagtcccg 60
   tgcttgttcg tgtgggtgag atagtaagtg caatct 96
<210> 12
   <211> 97
   <212> DNA
   <213> Künstliche Sequenz
<220>
   <223> Beschreibung der künstlichen Sequenz: Nukleotidsequenz
<400> 12
   ataccagctt attcaattcc aaaggtaaac cgcataataa gggtatgtat taaattgtgt 60
   ggtgatgact gatgccataa gatagtaagt gcaatct 97
<210> 13
   <211> 94
   <212> DNA
   <213> Künstliche Sequenz
<220>
   <223> Beschreibung der künstlichen Sequenz: Nukleotidsequenz
<400> 13
   ataccagctt attcaattga ggatcacctg ctctgccacc ctttttaacg tggggttaca 60
   tttgctgaag ggcttgagat agtaagtgca atct                              94
<210> 14
   <211> 96
   <212> DNA
   <213> Künstliche Sequenz
<220>
   <223> Beschreibung der künstlichen Sequenz: Nukleotidsequenz
<400> 14
   ataccagctt attcaattgt accagccgag atcttttttg acgatatgtg ttttttttga 60
   ggtgttgagt ttagtgtgag atagtaagtg caatct                        96
<210> 15
   <211> 77
   <212> DNA
   <213> Künstliche Sequenz
<220>
   <223> Beschreibung der künstlichen Sequenz: Nukleotidsequenz
<400> 15
   ataccagctt attcaattgc gataaatttt gctaagtgcg gtcaagactg tgttcgtgta 60
   gatagtaagt gcaatct                                                 77
<210> 16
   <211> 65
   <212> DNA
   <213> Künstliche Sequenz
<220>
   <223> Beschreibung der künstlichen Sequenz: Nukleotidsequenz
<400> 16
   ataccagctt attcaattgc cataccgtag ttagcatatg tagtgttaga tagtaagtgc 60
   aatct                                                              65
<210> 17
   <211> 96
   <212> DNA
   <213> Künstliche Sequenz
<220>
   <223> Beschreibung der künstlichen Sequenz: Nukleotidsequenz
<400> 17
   ataccagctt attcaattgc gcctttaaat ataacccgag tgctttgttt gaactggtgt 60
   tccggatggc ctgtgttgag atagtaatgt caatct                           96
<210> 18
   <211> 96
   <212> DNA
   <213> Künstliche Sequenz
<220>
   <223> Beschreibung der künstlichen Sequenz: Nukleotidsequenz
<400> 18
   ataccagctt attcaattgc ttgcatcgtc attatgaggt ggattcaact gtttttgact 60
   ttttgcccct ggacgctgag atagtaagtg caatct                           96
<210> 19
   <211> 95
   <212> DNA
   <213> Künstliche Sequenz
<220>
   <223> Beschreibung der künstlichen Sequenz: Nukleotidsequenz
<400> 19
   ataccagctt attcaattgg ttaccattct ggtgggaccc gtgttgcctg gatgtgtttt 60
   agtttttttg gtgttttaga tagtaagtgc aatct                            95
<210> 20
   <211> 95
   <212> DNA
   <213> Künstliche Sequenz
<220>
   <223> Beschreibung der künstlichen Sequenz: Nukleotidsequenz
<400> 20
   ataccagctt attcaattgg ttggagacct tattggcagc atgcagggcc ctcagctgtg 60
   caaccccggt ttccgttaga tagtaagtgc aatct                            95
<210> 21
   <211> 68
   <212> DNA
   <213> Künstliche Sequenz
<220>
   <223> Beschreibung der künstlichen Sequenz: Nukleotidsequenz
<400> 21
   ataccagctt attcaattca cacatgcgcc ttagttagcc ctggttgttg agatagtaag 60
   tgcaatct                                                           68